(19) Europäisches Patentamt
European Patent Office
Office européen des brevets

(11) Publication number: 0 234 609 B1

(12) EUROPEAN PATENT SPECIFICATION

(45) Date of publication of the patent specification: 29.08.90

(51) Int. Cl.5: **C07D 303/36**, C08G 59/32

(21) Application number: 87200067.4

(22) Date of filing: 16.01.87

# (54) Novel polyglycidyl amines.

(30) Priority: 22.01.86 US 821581

(43) Date of publication of application: 02.09.87 Bulletin 87/36

(45) Publication of the grant of the patent: 29.08.90 Bulletin 90/35

(84) Designated Contracting States: BE DE ES FR GB IT NL SE

(56) References cited:
EP-A- 0 131 281
EP-A- 0 241 931

CHEMICAL ABSTRACTS, vol. 105, no. 26, 29th December 1986, pages 38-39, abstract no. 227870u, Columbus, Ohio, US; & JP-A-61 97 276

(73) Proprietor: SHELL INTERNATIONALE RESEARCH MAATSCHAPPIJ B.V., Carel van Bylandtlaan 30, NL-2596 HR Den Haag(NL)

(72) Inventor: Schenk, Cornelis, Marienstien 166, NL-1852 SM Heiloo(NL)
Inventor: De La Mare, Harold Elison, 42 Chee Hoon, Singapore 1129(SG)
Inventor: Bauer, Ronald Sherman, 5427 Crown Colony, Houston Texas 77069(US)

(74) Representative: Aalbers, Onno et al, P.O. Box 302, NL-2501 CH The Hague(NL)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 234 609 B1

ACTORUM AG

## Description

This invention relates to novel polyglycidyl amines and to their use in the preparation of curable thermosetting resin systems.

Thermosetting compositions containing epoxy resins and reinforcing fibres have been found highly suitable for applications requiring high-strength, lightweight structural materials. One drawback in the use of conventional high-performance epoxy resins having a Tg of at least 200 °C for high-performance structural materials suitable for automotive and aerospace applications is the tendency of the epoxy matrix material to absorb water. The effect of absorbed water is a decrease in the high-temperature properties of the structural material. Exposure to humid environments can depress the glass transition temperature of epoxy resins by as much as 20-25 °C/weight per cent of water absorbed. Absorbed water can cause loss of elastic modulus and other performance properties of epoxy resins as well.

It is therefore an object of the present invention to provide novel thermosetting materials having low water absorption. To this end the invention provides certain resin components, which are novel compounds.

According to the invention, novel glycidated aromatic amines are provided which have the following chemical structure:

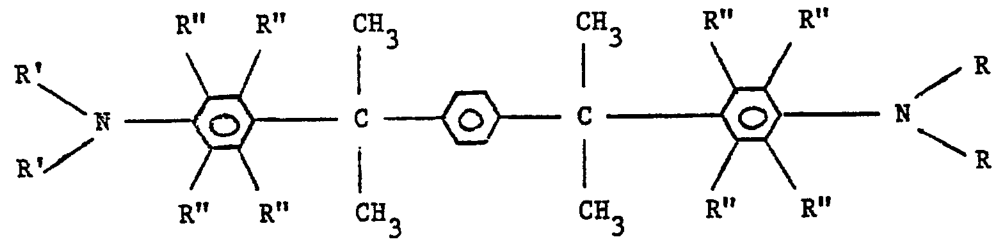

in which each R and each R′ is selected independently from H, $CH_3$ and $CH_2CH_3$ and at least one R and one R′ is

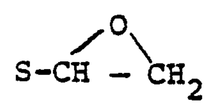

wherein each S is selected independently from $-CH_2-$ and $-CH_2CH_2-$, and each R″ is selected independently from H, $C_1$-$C_{10}$ alkyl and halide.

The present novel glycidylated aromatic amines are particularly suitable for use in reinforced epoxy resin systems which can be used for applications such as automotive and aerospace materials fabrication. Currently preferred curing agents for such systems are aromatic diamines. The cured resins have improved resistance to water absorption.

Preferred glycidylated aromatic amines are those of the above formula wherein each R″ is selected independently from H, $CH_3$ and F, and each R′ and each R is

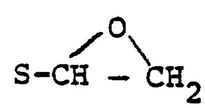

wherein each S is $CH_2$. Such preferred glycidylated aromatic amines include compounds having the structure

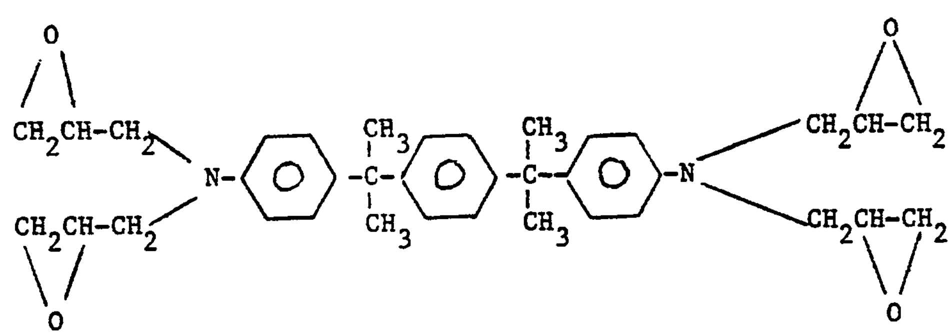

tetraglycidyl-α,α′-bis(4-aminophenyl)-p-diisopropylbenzene, a solid tetraglycidyl amine having a glass transition temperature (uncured) of 23 °C, and

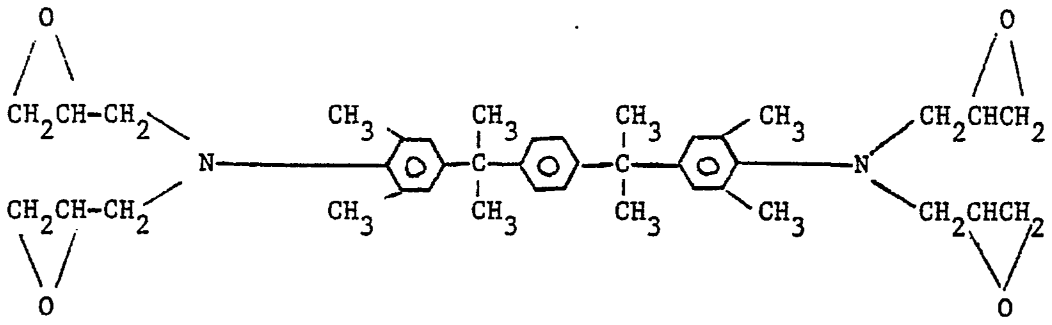

tetraglycidyl-α,α′-bis(3,5-dimethyl-4-aminophenyl)-p-diisopropylbenzene, a solid tetraglycidyl amine having a glass transition temperature (uncured) of 41 °C.

One or more R″ can be halide, as might be desirable when flame retardancy is needed. Halide R″ is preferably chlorine, fluorine or bromine. The preparation of tetraglycidyl-α,α′-bis(3-fluoro-4-aminophenyl)-p-diisopropenylbenzene is illustrated in Examples 5 and 6.

The glycidylated aromatic amines can be prepared by reacting the corresponding polyaromatic amine with epichlorohydrin, as illustrated in Examples 1, 2, 4 and 6. The polyaromatic amine can be prepared by reacting the corresponding aniline with diisopropylbenzene in the presence of hydrochloric acid or acid clay catalyst, as illustrated in Examples 3 and 5.

The novel thermosetting compositions contain a curing agent in addition to the above glycidylated aromatic amines. Effective curing agents include, for example, amines, acids, anhydrides and imidazoles. The preferred curing agents for imparting good strength, water resistance and high temperature resistance to the composition are substituted or unsubstituted aromatic amines. The aromatic amines are preferably aromatic diamines and triamines such as, for example, methylene dianiline, m-phenylene diamine, α,α′-bis(3,5-dimethyl-4-aminophenyl)-p-diisopropenylbenzene and blends of aromatic diamines available commercially from Shell Chemical Company as EPON® Curing Agents Y and Z. The most preferred curing agent because of the superior heat resistance of the resulting composition is diaminodiphenylsulfone. Such a curing agent is available as Sumicure S® from Sumitomo. Other very preferred curing agents are methylene dianiline and m-phenylene diamine.

The curing agent will be present in the composition in an amount effective to cure thermosettable components of the composition. Generally, the curing agent will be present in an amount of from 0.2 to 1.5 equivalents per equivalent of thermosetting components, usually from 0.4 to 1.3 equivalents. In terms of weight per cent, the preferred diaminodiphenylsulfone curing agent will generally be present in an amount of 6 to 50, usually 10 to 50, preferably 15 to 45 weight per cent, based on the weight of the resin/curing agent composition. The curing conditions for the glycidylated aromatic amines can vary widely depending upon the curing agent used and the properties desired in the cured composition. Curing conditions using diaminodiphenylsulfone, for example, will generally include heating the resin to a temperature of about 170-200 °C for 2-4 hours.

The composition can also include one or more additional thermosetting or thermoplastic components, such as functionalized elastomers, bismaleimides and epoxy resins. The presently preferred composition contains a blend of the above-described glycidylated aromatic amines and epoxy resins.

The epoxy resin component, when present in the invention compositions can be any curable epoxy resin having, on the average, more than one epoxide group per molecule. The epoxy resin can be saturated or unsaturated, aliphatic, cycloaliphatic, aromatic or heterocyclic, and may bear substituents which do not materially interfere with the curing reaction. They may be monomeric or polymeric.

Suitable epoxy resins include glycidyl ethers prepared by the reaction of epichlorohydrin with a compound containing at least one hydroxyl group carried out under alkaline reaction conditions. The epoxy resin products obtained when the hydroxyl group-containing compound is bisphenol-A are represented below by structure I wherein n is zero or a number greater than 0, commonly in the range of 0 to 10, preferably in the range of 0 to 2.

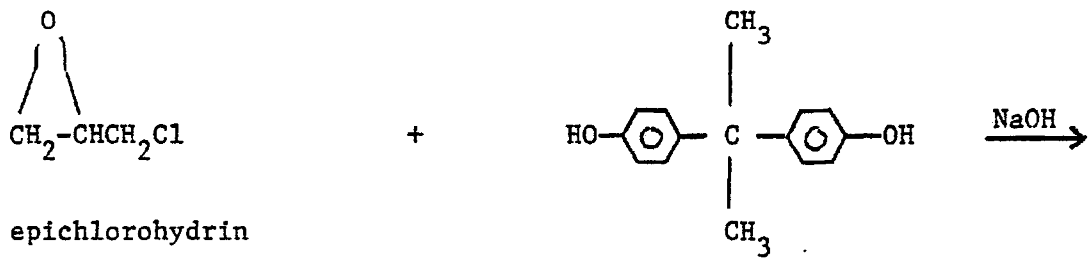


epichlorohydrin

bisphenol-A

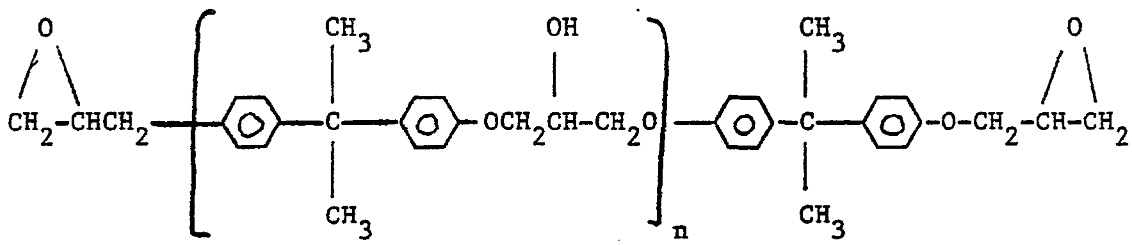

I

Other suitable epoxy resins can be prepared by the reaction of epichlorohydrin with mononuclear di- and trihydroxy phenolic compounds such as resorcinol and phloroglucinol, selected polynuclear polyhydroxy phenolic compounds such as bis(p-hydroxyphenyl)methane and 4,4′-dihydroxybiphenyl, or aliphatic polyols such as 1,4-butanediol and glycerol.

Epoxy resins particularly suitable for blending in the invention compositions have molecular weights generally within the range of 50 to 10,000, preferably 200 to 1500. The commercially available epoxy resin EPON® Resin 825, a reaction product of epichlorohydrin and 2,2-bis(4-hydroxyphenyl)propane (bisphenol-A) having a molecular weight of 400, an epoxide equivalent (ASTM D-1652) of about 172-178, and an n value in formula I above of 0, is a preferred epoxy resin blending component because of its commercial availability and the processing characteristics imparted to the resulting composition.

The novel glycidylated aromatic amine and the epoxy resin or other thermosettable component, if present, can be combined in any amounts which impart the desired properties to the cured or uncured resin. Blends of the thermosetting components will generally be expected to fall within the range of weight ratios of glycidylated aromatic amine to second component of 5:95-95:5, preferably 25:75-75:25, most preferably 35:65-65:35. Weight ratios of glycidylated aromatic amine to epoxy resin of 40:60 to 60:40 have been found particularly suitable for high-performance applications.

Mixing of the components of the composition is conveniently carried out by melt blending the novel glycidylated aromatic amine and the epoxy resin at temperatures of from 80 °C to 150 °C and then adding the curing agent with mixing.

The composition optionally, but preferably for high-performance applications such as automotive and aerospace, contains a reinforcing substrate. Suitable reinforcing materials include, for example, glass fibres, carbon fibres, polyaramide, boron, calcium carbonate, talc, alumina or asbestos. The preferred fibrous reinforcing material for high-performance applications is continuous carbon fibre. The fibrous reinforcing material will be present in the composition in an amount effective to impart increased strength to the cured composition, generally from 40 to 95 weight per cent, usually from 60 to 80 weight per cent, based on the weight of the total composition.

The glycidylated aromatic amine can be applied to the fibrous reinforcing material from the melt or solution by methods known in the art. The glycidylated aromatic amine/curing agent-impregnated substrate, or "prepreg," or a laminate prepared from a plurality of prepregs, is then cured, generally at a temperature of from 160 °C to 300 °C for 30 minutes to 4 hours and a pressure of 160 to 240 psi, to form the structural composite article.

The invention composition can optionally include additives for control or modification of various properties of the composition in its cured or uncured state, including cure rate accelerators or retardants, tackifiers and the like.

The glycidylated aromatic amines are useful as curable components of coatings, adhesives and structural composites.

EXAMPLE 1

This example illustrates the preparation of tetraglycidyl-α,α'-bis(4-aminophenyl)-p-diisopropylbenzene.

Into a 5 litre, 4-neck, round bottom flask, equipped with a stirrer, condenser, thermocouple, addition funnel and nitrogen sweep, was charged 250 g of α,α'-bis(4-aminophenyl)-p-diisopropenylbenzene, 1345.4 g of epichlorohydrin, 366.4 g of water, and 873.9 g of isopropyl alcohol. While stirring, the reaction mixture was heated to 80 °C and held for 2 hours. 684.2 g of 20% sodium hydroxide was added dropwise over 7 minutes. The reaction was held at 82 °C for 2 hours and 20 minutes. The reaction mixture formed two phases, which were allowed to separate. The lower phase was drained and analyzed for per cent base. The reaction was continued until the total base was equal to or less than 0.1 %wt. The isopropyl alcohol and epichlorohydrin were removed from the upper phase and the resulting solid was dissolved in methylisobutyl ketone to make a 30 %wt solution. This mixture was returned to the reaction flask. 342.1 g of 20 %wt sodium hydroxide was added to the mixture which was then brought to 80 °C and held for 2 hours. The two phases were again allowed to separate and the lower phase drained. The upper phase was washed 3 times with 900 ml of hot (90 °C) water. The methylisobutyl ketone was then removed yielding 342.7 g solid or 83.0%. The weight per epoxide (WPE) was 155 and the saponifiable chlorine was 0.04 %wt. The melting point is 65 °C.

EXAMPLE 2

This example illustrates the preparation of tetraglycidyl-α,α'-bis(3,5-dimethyl-4-aminophenyl)-p-diisopropylbenzene.

Into a 500 ml, 4-neck, round bottom flask, equipped with stirrer, condenser, heat, addition funnel, thermocouple and nitrogen sweep was charge 300 g of the α,α'-bis(3,5-dimethyl-4-aminophenyl)-p-diisopropenylbenzene, 974 g of isopropyl alcohol, 1665.6 g epichlorohydrin, and 291 g of water. While stirring, it was heated to reflux. 720 g of 20 %wt sodium hydroxide was added dropwise over four hours. The reaction mixture was then held an additional hour at 82 °C. The two phases were allowed to separate and the lower phase was drained and analyzed for per cent base. The reaction was continued until the total base was equal to or less than 0.1 %wt. The epichlorohydrin and isopropyl alcohol were removed from the upper phase the resulting solid dissolved in 1000 mls of methylisobutyl ketone. The mixture was returned to the reaction flask and 1000 mls of 5% sodium hydroxide was added to the mixture which was heated to 89 °C and held for 3 hours. The two phases were allowed to separate and the lower layer analyzed for per cent base. The brine was drained and the upper phase was washed 3 times with 1000 mls of hot water. The methylisobutyl ketone was removed yielding 387 g or 82.4%. WPE was 181 and the saponifiable chlorine was 0.028 %wt. Melting point is 51 °C.

EXAMPLE 3

This example illustrates the preparation of α,α'-bis(3-methyl-5-ethyl-4-aminophenyl)-p-diisopropylbenzene.

Into a 3 litre, 3-neck round bottom flask, equipped with a stirrer, dean-stark trap, condenser, thermocouple, and nitrogen sweep, was charged 2087.6 g of 2-methyl-6-ethylaniline, 300 g 1,4-bis(hydroxyisopropylphenyl)benzene (p-diol) and 93 g of Filtrol Grade No. 1 (Harshaw Chemical Company). While stirring, the reaction mixture was heated to 160 °C removing any water of dehydration with the dean-stark trap, and held for 2 hours. The mixture was filtered hot to remove the filtrol and the aniline was removed under vacuum. Yield was 560.7 g or 84.7%.

EXAMPLE 4

The example illustrates the preparation of tetraglycidyl-α,α'-bis(3-methyl-5-ethyl-4-aminophenyl)-p-diisopropylbenzene.

Into a 5000 ml, 4-neck round bottom flask, equipped with a stirrer, condenser, thermocouple, addition funnel and nitrogen sweep was charged 300 g of the amine prepared in Example 3, 1289.7 g of epichlorohydrin, 841.1 g of isopropyl alcohol, and 252.3 g of water. The mixture was heated to 80 °C with stirring and was held for 2 hours. 672.9 g of 20% sodium hydroxide was added dropwise over 4 hours. The phases were allowed to separate and the epichlorohydrin and isopropyl alcohol were removed from the upper phase; the resulting solid dissolved at 30 %wt in methylisobutyl ketone. The mixture was returned to the reaction flask and 336.5 g of 20 %wt sodium hydroxide was added to the mixture which was then heated to 85 °C and held for 2 hours. The two phases were allowed to separate and the brine was drained and the upper phase was washed 3 times with water. The methylisobutyl ketone was removed yielding 434.6 g or 95.1%. WPE was 196 and the saponifiable chlorine was 0.007 %wt.

EXAMPLE 5

This example illustrates the preparation of α,α'-bis(3-fluoro-4-aminophenyl)-p-diisopropylbenzene.

Into a 1 litre, 3-neck round bottom flask, equipped with a stirrer, dean-stark trap, condenser, thermocouple, and nitrogen sweep, was charged with 500 g of o-fluoroaniline and 25 g of Filtrol Grade No. 1. While stirring, the reaction mixture was heated to 133 °C to drive off any water. The mixture was cooled to 30 °C and 72.7 g of p-diol charged. While stirring it was heated to 170 °C and held for 3 hours. More water was collected. The mixture was filtered hot to remove the filtrol, chilled in an ice bath, and filtered again yielding 81.0 g, melting point 148-150 °C. The filtrate was vacuum distilled to remove the o-fluoroaniline (286 g recovered). The resulting solid was recrystalized from toluene/hexane yielding 48.5 g. The total yield was 129.5 g or 90.9%.

EXAMPLE 6

This example illustrates the preparation of tetraglycidyl-α,α'-bis(3-fluoro-4-aminophenyl)-p-diisopropylbenzene.

Into a 2000 ml, 4-neck round bottom flask, equipped with a stirrer, condenser, thermocouple, addition funnel and nitrogen sweep was charged 95 g of the amine prepared in Example 5, 555.2 g of epichlorohydrin, 409.2 g of isopropyl alcohol, and 175.4 g of water. The mixture was heated to 80 °C with stirring and was held for 4 hours. 240 g of 20% sodium hydroxide was added dropwise over 1 hour and 45 minutes. The mixture was held an additional 30 minutes at which time the phases were allowed to separate and the lower phase was analyzed for per cent base (0.15%). The epichlorohydrin and isopropyl alcohol were removed from the upper phase and the resulting solid dissolved at 30 %wt in methylisobutyl ketone. The mixture was returned to the reaction flask and 120 g of 20 %wt sodium hydroxide was added to the mixture which was then heated to 85 °C and held for 1 hour. The two phases were allowed to separate and the lower layer analyzed for per cent base (12.1%). The brine was drained and the upper phase was washed 4 times with 500 mls of hot water. The methylisobutyl ketone was removed yielding 139.2 g or 92.2%. WPE was 173 and the saponifiable chlorine was 0.0899 %wt.

EXAMPLES 7 to 10

Four epoxy resin compositions were prepared comprising the following components in parts by weight given in Table 1:

Table 1

| | Example 7 | Example 8 | Example 9 | Example 10 |
|---|---|---|---|---|
| TGMDA* | 100 | --- | --- | --- |
| GLYCIDYL AMINE | | | | |
| Example 1 | --- | 100 | --- | --- |
| Example 2 | --- | --- | 100 | --- |
| Example 6 | --- | --- | --- | 100 |
| SU-8* | 8.2 | 8.2 | 8.2 | 8.2 |
| DDS* | 51.9 | 42.4 | 36.6 | 38.2 |

* for comparison

The TGMDA was tetraglycidyl-4,4'-diaminodiphenylmethane sold by Ciba-Geigy under the tradename CIBA MY-720, the SU-8 is a polyfunctional epoxy hard resin sold by Celanese Polymer Specialties Company, and the DDS was a 4,4'-diaminodiphenylsulfone sold by Sumitomo Chemical Company under the trade name Sumicure S. The four glycidyl amine compositions described in Table 1 were cast into test specimens accordingly:

A. 50 g of MY-720 was weighed into a beaker with 4.1 g of SU-8 and placed in an oven to melt at 150 °C. 25.9 g of DDS was heated to 150 °C and added to the resin with hand mixing. The mixture was placed back into the 150 °C oven for 23 minutes, with frequent stirring, until the DDS was dissolved. The mixture was then degassed in a hot vacuum oven for 3 minutes and poured into a preheated glass mould. The plaque was cured for 2 hours at 150 °C, then 4 hours at 200 °C.

B. 50 g of the tetraglycidyl amine prepared in Example 1 was weighed into a beaker with 4.1 g of SU-8 and heated to 150 °C until melted. 21.2 g of hot (150 °C) DDS was added to the hot resin mixture with hand mixing. It was placed back into the oven for approximately 22 minutes, mixing every few seconds. The mixture was degasses in a hot vacuum oven for 15 minutes, then poured into a preheated glass mould. The plaque was cured for 2 hours at 150 °C, then 4 hours at 200 °C.

C. 50 g of the tetraglycidyl amine prepared in Example 2 was weighed into a beaker with 4.1 g of SU-8 and heated to 170 °C until melted. 18.3 g of DDS was heated to 170 °C and added to the resin mixture with hand mixing. It was placed back into the oven for 35 minutes with frequent stirring. After the DDS was in solution, the mixture was degassed in a hot vacuum oven for 3 minutes and then poured into a hot mould. The plaque was cured for 2 hours at 150 °C and four hours at 200 °C.

D. 50 g of the tetraglycidyl amine prepared in Example 6 was weighed into a beaker with 4.1 g of SU-8 and heated to 150 °C in an oven to melt. 19.1 g of DDS was heated to 150 °C and added to the resin with hand mixing. It was held at 150 °C for 22 minutes with frequent mixing. The resin mixture was degassed in a hot vacuum oven for 3 minutes and then poured into a glass mould. The plaque was cured at 150 °C for 2 hours and 200 °C for 4 hours.

The glass transition temperature, flex properties, fracture toughness and water absorption were determined for each composition. Glass transition temperatures were obtained on a PERKIN-ELMER DSC-4 Differential Scanning Calorimeter, the flex strength and flex modulus were obtained following ASTM D790M-81, Method 1, and fracture toughness was obtained by a method based on ASTM E399-83. Water absorption was determined after two weeks immersion at 93 °C.

The results given in Table 2 show that the water absorption of the compositions according to the present invention is approximately half or less of that of the corresponding TGMDA system and the retention of the modulus under hot/wet conditions is also improved over the TGMDA system. The table also shows that the other properties of the cured resin compositions are not significantly different from those desired for matrix resin compositions for advanced composites.

Table 2

| PROPERTIES | Example 7 | Example 8 | Example 9 | Example 10 |
|---|---|---|---|---|
| Tg (DSC), °C | 262 | 237 | 242 | 225 |
| FLEX PROPERTIES, (DRY)1/ | | | | |
| STRENGTH, KSI | 19 | --- | 10.1 | 10.9 |
| MODULUS, KSI | 549.0 | 581.0 | 539.0 | 516.7 |
| ELONGATION, % | 4.7 | --- | 2.1 | 2.3 |
| FLEX PROPERTIES, (WET)2/ | | | | |
| STRENGTH, KSI | 10.8 | 9.9 | 12.0 | 11.5 |
| MODULUS, KSI | 351.8 | 512.3 | 471.2 | 434.7 |
| ELONGATION, % | 3.7 | 2.2 | 2.9 | 3.0 |
| FRACTURE TOUGHNESS | | | | |
| Kq, psi in$^2$ | 1027 | 547 | 821 | 811 |
| MOISTURE GAIN | 5.8 | 3.3 | 2.5 | 2.8 |

1/ Test performed at room temperature

2/ Tested in water at 93 °C after 2 weeks immersion at 93 °C

## Claims

1. A novel glycidylated aromatic amine having the formula

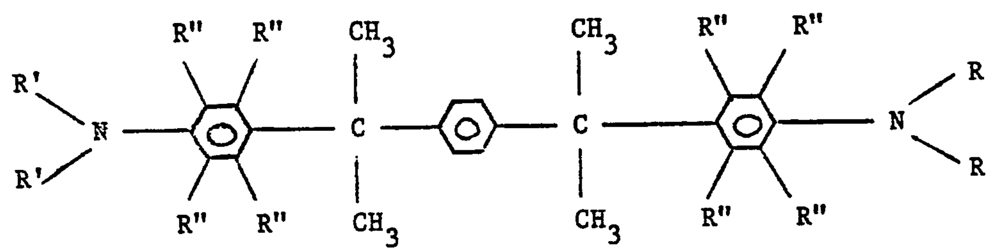

wherein each R and each R′ is selected independently from H, $CH_3$, $CH_2CH_3$ and at least one R and one R′ is

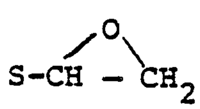

wherein each S is selected independently from $CH_2$ and $CH_2CH_2$, and each R″ is selected independently from H, $C_1$-$C_{10}$ alkyl and halide.

2. A compound as claimed in claim 1 in which each R″ is H.

3. A compound as claimed in claim 1 in which each R and each R′ is

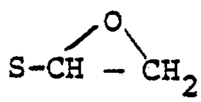

4. A compound as claimed in claim 3 in which each R″ is H.
5. A compound as claimed in claim 1 in which each S is $CH_2$.
6. A compound as claimed in claim 1 in which at least two R″ are $CH_3$.
7. A compound as claimed in claim 1 in which at least one R″ is selected from chlorine, bromine and fluorine.
8. Tetraglycidyl-α,α′-(4-aminophenyl)-p-diisopropylbenzene.
9. A thermosetting resin system, comprising an epoxy resin, a curing agent and a glycidylated aromatic amine as claimed in any one of claims 1 to 8.
10. The composition of claim 9 in which the epoxy resin is a polyglycidyl ether having a molecular weight within the range of from 200 to 1500.
11. The composition of claim 9 or 10, in which the glycidylated aromatic amine and the epoxy resin are present in a weight ratio of from 40:60 to 60:40.

**Patentansprüche**

1. Neues glycidyliertes aromatisches Amin der Formel

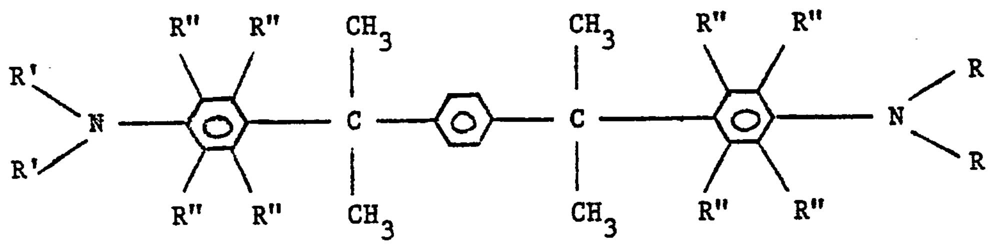

worin jedes R und jedes R′ unabhängig voneinander ausgewählt ist aus H, $CH_3$, $CH_2CH_3$ und mindestens ein R und ein R′

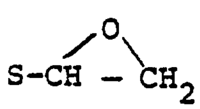

ist, worin jedes S unabhängig voneinander ausgewählt ist aus $CH_2$ und $CH_2CH_2$ und jedes R″ unabhängig voneinander ausgewählt ist aus H, $C_1$–$C_{10}$ Alkyl und Halogenid.
2. Verbindung nach Anspruch 1, worin jedes R″ gleich H ist.
3. Verbindung nach Anspruch 1, worin jedes R und jedes R′ gleich

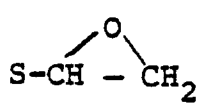

ist.
4. Verbindung nach Anspruch 3, worin jedes R″ gleich H ist.
5. Verbindung nach Anspruch 1, worin jedes S gleich $CH_2$ ist.
6. Verbindung nach Anspruch 1, worin mindestens zwei R″ gleich $CH_3$ ist.
7. Verbindung nach Anspruch 1, worin mindestens ein R″ ausgewählt ist aus Chlor, Brom oder Fluor.
8. Tetraglycidyl-α,α′-(4-aminophenyl)-p-diisopropylbenzol.
9. Wärmehärtbares Harzsystem, umfassend ein Epoxyharz, ein Vernetzungsmittel und ein glycidyliertes aromatisches Amin nach einem der Ansprüche 1 bis 8.
10. Zusammensetzung nach Anspruch 9, worin das Epoxyharz ein Polyglycidylether mit einem Molekulargewicht im Bereich von 200 bis 1500 ist.
11. Zusammensetzung nach Anspruch 9 oder 10, worin das glycidylierte aromatische Amin und das Epoxyharz in einem Gewichtsverhältnis von 40:60 bis 60:40 vorliegen.

## Revendications

1. Une nouvelle amine aromatique glycidylée ayant la formule

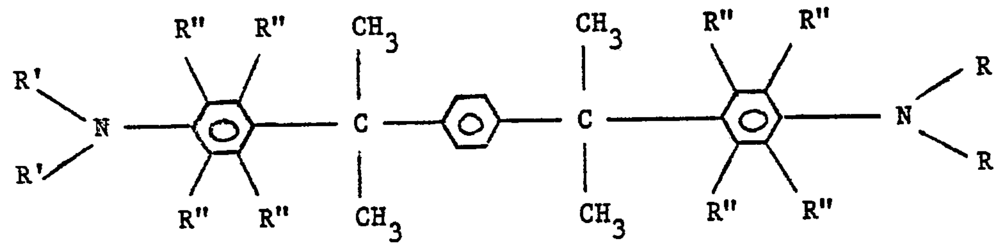

où chaque R et chaque R′ sont choisis indépendamment parmi H, $CH_3$, $CH_2CH_3$ et au moins un R et un R′ sont chacun

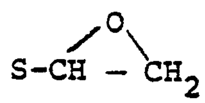

où chaque S est choisi indépendamment parmi $CH_2$ et $CH_2CH_2$ et chaque R″ est choisi indépendamment parmi H, des groupes alcoyle en $C_1$–$C_{10}$ et les halogènes.

2. Un composé selon la revendication 1, dans lequel chaque R″ est H.

3. Un composé selon la revendication 1, dans lequel chaque R et chaque R′ sont

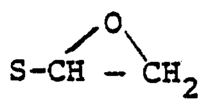

4. Un composé selon la revendication 3, dans lequel chaque R″ est H.

5. Un composé selon la revendication 1, dans lequel chaque S est $CH_2$.

6. Un composé selon la revendication 1, dans lequel au moins deux R″ sont $CH_3$.

7. Un composé selon la revendication 1, dans lequel au moins un R″ est choisi parmi le chlore, le brome et le fluor.

8. Le tétraglycidyl-alpha,alpha′-(4-aminophényl)-p-diisopropylbenzène.

9. Un système de résine thermodurcissable, comprenant une résine époxy, un agent réticulant et une amine aromatique glycidylée selon l'une quelconque des revendications 1 à 8.

10. La composition selon la revendication 9, dans laquelle la résine époxy est un éther polyglycidique ayant un poids moléculaire compris entre 200 et 1500.

11. La composition selon la revendication 9 ou 10, dans laquelle l'amine aromatique glycidylée et la résine époxy sont présentes dans un rapport en poids compris entre 40:60 et 60:40.